# EUROPEAN PATENT APPLICATION

(11) **EP 4 582 802 A2**
(43) Date of publication of application: **09.07.2025**
(21) Application number: 25171971.2
(22) Date of filing: 08.06.2022
(51) Int. Cl.: G01N 33/24

(54) **DIGITAL PARTICLE ANALYSIS**

(30) Priority: 08.06.2021 WO PCT/EP2021/065367; 24.01.2022 EP 22153044
(62) Divisional of application: 22731712.0
(71) Applicant: Sika Technology AG, 6340 Baar (CH)
(72) Inventor: VELTEN, Ulf, 8048 Zürich (CH); APEH, Christopher, 8048 Zürich (CH); KUNDRA, Milan, Hertfordshire, AL7 1BQ (GB); LINDLAR, Benedikt, 8048 Zürich (CH); LISKA, Martin, Hertfordshire, AL7 1BQ (GB); MARAZZINI, Oscar, 20068 Peschiera Borromeo (IT); OBST, Stefan, 8048 Zürich (CH); RIEGER, Carsten, 8048 Zürich (CH); VOGELSANG, Jörg, 8048 Zürich (CH); VORWERK, Michael, 8048 Zürich (CH); WEINKAUF, Annette, 8048 Zürich (CH); ZIMMERMANN, Jörg, 8048 Zürich (CH)
(74) Representative: Sika Patent Attorneys

(57) **Abstract**

A computer-implemented method for characterization of solid particles, especially sand particles, comprises the steps of:
a) Providing a sample of solid particles to be analyzed in a predefined sample area;
b) Taking at least one digital image of the sample of solid particles with a camera of a mobile computer device;
c) Performing an imaging particle analysis of the at least one digital image for extracting at least one particle size parameter and/or at least one particle shape parameter of the population of particles identified in the at least one digital image;
d) Making available the at least one particle size parameter and/or the at least one particle shape parameter via a user interface, via a machine interface and/or on a data storage medium.

## Description

### Technical field

The invention relates to a computer-implemented method for characterization of solid particles, especially sand particles, as well as systems with means to carry out the method, and a computer-readable medium comprising instructions for carrying out the method. Further aspects of the invention are related to methods for providing a formulation of a curable composition and a method for producing a curable composition.

### Background art

In construction industry, curable compositions are widely used for various applications. Examples of such compositions are mortar, concrete, grout, or screed compositions. These compositions typically comprise binders in combination with solid aggregates.

Binders might be selected from mineral binders, such as e.g. hydraulic binders, as well as organic binders, e.g. curable polymer compositions. Aggregates are chosen depending on the desired properties of the curable compositions. Typically aggregates include sand, gravel, rock flour, and/or polymeric particles.

Regarding sands, for example, usually high-quality river sands have been used up to now. In the future, however, high-quality sands will become rare and hard to obtain. Thus, more and more low-quality sands have to be used, either from natural sources, e.g. low-grade river sands, or from special manufacturing processes, e.g. crushed rocks. This will have a huge impact on the formulations of curable compositions, especially concrete and mortar compositions, since the specific nature of the sands have a significant impact on the properties of the curable compositions. This has to be taken into account properly because, despite the decreasing sand quality, the technical requirements for cured compositions, e.g. hardened concrete or mortar, will remain unchanged or even be raised.

In general, in order to produce curable compositions with desired properties in a targeted manner, it is important to take into account the specific nature of the aggregates. Regarding low-quality sands, for example, it is in particular important to check if with the low-quality sand (i) the nature of fines is critical, (ii) the particle shape is critical and/or if (iii) the fines content is sufficient.

Specifically, the behavior of sand in curable compositions, especially concrete and mortar compositions, depends inter alia on the crushing technique, particle shape, surface texture, fines content, mineral contamination, and grading curve.

Grading curves of sand and aggregates is a key element to calculate the grading of a concrete mix design to ensure a dense packing of sand and aggregates. Thereby, in particular, the particle shape, which in turn depends on the production method or the sand and/or aggregate source, typically influences the needed grading of the concrete mix design.

For manufactured sand, especially items (ii) and (iii) are relevant. The fines content can be determined via sieve analysis. Although it is a standard procedure, it is very time consuming. Sieving shows if the grading curve is as desired or if it has to be modified by mixing with other sand fractions.

In contrast, the particle shape, i.e. the above-mentioned item (ii), is hardly analyzed in daily work in order to optimize the grading curve of a curable composition. This is normally only done if it is needed to control whether the aggregates comply with certain standards. This is due to the fact that analyzing the particle shape of a specific sand is very time consuming and needs special equipment, for example as defined in standard EN 933-1:2012 to -7:2012.

Thus, there is still a need to provide improved solutions which allow for comprehensively characterizing aggregates.

### Disclosure of the invention

It is an object of the present invention to provide improved solutions for characterizing aggregates. Aggregates include in particular sands. Thereby, preferably, the solutions should allow for a fast and reliable characterization process with a minimum of equipment required. Especially preferred, the solutions should allow for performing a complete characterization of aggregates in daily practice, in particular with regard to grading curve and their particle shape parameters. Preferably such that with the characterized aggregates, formulations of curable compositions with desired properties can be produced in a targeted manner.

Surprisingly, it has been found that the features of claim 1 achieve this object. Thus, the core of the present invention is related to a computer-implemented method for characterization of solid particles, especially sand particles, comprising the steps of:
a) Providing a sample of solid particles to be analyzed in a predefined sample area, especially a two-dimensional sample area;
b) Taking at least one digital image of the sample of solid particles with a camera of a mobile computer device or a camera connected to a mobile device;
c) Performing an imaging particle analysis of the at least one digital image for extracting at least one particle size parameter and/or at least one particle shape parameter, preferably at least one particle size parameter and at least one particle shape parameter, of the population of particles in the at least one digital image;
d) Making available the at least one particle size parameter and/or the at least one particle shape parameter, preferably the at least one particle size parameter and the at least one particle shape parameter, via a user interface, via a machine interface and/or on a data storage medium.

The inventive method is a unique approach which can be implemented on conventional mobile devices, such as e.g. smartphones. Thus, there is no need for complex and expensive analysis equipment. The method furthermore allows for a very fast, flexible and accurate digital characterization of solid particles, both in terms of size and shape. Thereby, solid particles of different nature and size can easily be characterized with one and the same hardware devices.

Thanks to the fast and easy way to carry out characterization of solid particles, daily mix design optimization for curable compositions, e.g. for concrete compositions, is possible with neglectable additional effort.

Specifically, the method can be implemented in various ways, e.g. in the form of a standalone application running on the mobile device without need for any further resources such as for example server systems. This is in particular helpful in areas with limited access to communication networks, e.g. far away from urban centers or underground. However, the method can be implemented as well in a distributed computing environment, e.g. comprising the mobile device as a client in combination with a dedicated server as a storage unit and a specialized processing unit.

Also, the inventive method can be implemented in a flexible manner with known software architectures, e.g. as native application, a progressive web application (PWA), or a hybrid application (combination of native and PWA). Thereby, helpful internet links to tutorials or support sites as well as sharing functions (e.g. via e-mail, Bluetooth, Airdrop, or others communication means) can be included in such applications as well. Also, the inventive method can be implemented in one single application or it may be divided into two or more separate applications with appropriate software interfaces for data exchange between the applications. Furthermore, the application(s) can be extended with additional functions in a flexible manner.

Applications can be implemented for any kind of operating systems such as e.g. iOS, Android, Microsoft Windows and/or Linux.

The applications can be made available easily for anyone via different distribution channels, such as e.g. public download centers (for example Apple^{®} Store and Google Play^{®}) private company operated websites and/or dedicated download sites.

Additional aspects of the invention are subject of further independent claims. Particularly preferred embodiments are outlined throughout the description and the dependent claims.

### Ways of carrying out the invention

A first aspect of the present invention is directed to a computer-implemented method for characterization of solid particles, especially sand particles, comprising the steps of:
a) Providing a sample of solid particles to be analyzed in a predefined sample area;
b) Taking at least one digital image of the sample of solid particles with a camera of a mobile computer device or a camera connected to a mobile computer device;
c) Performing an imaging particle analysis of the at least one digital image for extracting at least one particle size parameter and/or at least one particle shape parameter, preferably at least one particle size parameter and at least one particle shape parameter, of the population of particles in the at least one digital image;
d) Making available the at least one particle size parameter and/or the at least one particle shape parameter, preferably at least one particle size parameter and at least one particle shape parameter, via a user interface, via a machine interface and/or on a data storage medium.

In the present context, a mobile computer device in particular is meant to be a handheld computer, i.e. a computer small enough to hold and operate in the hand of a human.

Especially the mobile computer device comprises a human interface device, especially comprising an input device and a display, and, preferably a communication interface, most preferably a wireless communication interface.

The mobile computer device in particular is selected from a mobile phone, a mobile computer or a portable computer. Especially, the mobile computer device is selected from a smartphone, a phablet, a tablet computer, a portable computer, a smartwatch, and/or a head-mounted display with camera. Highly preferred are mobile phones and/or smartphones.

Mobile phones and smartphones typically are equipped with high resolution cameras, an input device and a display. Thereby the input device and the display typically are combined in a touch sensitive display. Therefore, mobile phones and smartphones provide all of the hardware components required for performing the inventive method. Furthermore, such kind of devices can be held stably in the hand, which makes them highly suitable for taking images. At the same time mobile phones and smartphones typically have displays that are large enough for displaying complex data in well readable manner.

The expression "a camera of a mobile computer device" is meant to be an internal camera, which is integrated in the mobile computer device. In contrast, "a camera connected to a mobile computer device" means an external camera, which is a separate device connected to the mobile computer device. For example, the external camera is a camera attachable to the mobile computer device or a standalone camera. The connection between the external camera and the mobile computer device can be a wired and/or a wireless connection.

Preferably, the camera is a camera for taking images in the visible spectrum, especially color images. Color images allow for considering color properties of the solid particles and/or the predefined sample area in step c) of the inventive method. Thus, preferably, the at least one digital image taken in step b) is a color image. However, if color information is not required, other cameras, e.g. monochromatic cameras, can be used as well. In this case, the image is a monochromatic image, e.g. a black-and-white image.

Also, it is possible to use a camera for taking images outside the visible spectrum, e.g. in the infrared and/or ultraviolet range of the electromagnetic spectrum. Such cameras can be used alternatively or in addition to other cameras. In this case the properties of the solid particles in spectral ranges outside the visible spectrum can be considered in step c).

Preferably, the camera has a resolution of at least 2 megapixels, especially at least 5 megapixels, preferably at least 8 megapixels, particularly at least 12 megapixels, highly preferred at least 20 megapixels, even more preferred at least 50 megapixels or at least more than 100 megapixels. Especially, the camera has a resolution of at least 3'800 pixels × at least 2'100 pixels or a so called 4K, preferably 8K, more preferably 12K, especially 16K resolution. The higher the resolution the smaller solid particles can be identified in the sample area. However, for special embodiments, cameras with lower resolutions might be suitable as well.

Especially, the mobile computer device is configured for automatically recognizing the camera resolution.

Especially, a size of the optical sensor of the camera is at least 1/3", in particular at least 1/2.5", preferably at least 1/1.7", preferably at least 2/3", particularly at least 1/1.33" or at least 1/1.2". Particularly, a size of the optical sensor of the camera is from 1/3" to 1".

Put differently, a size of the optical sensor of the camera in terms of width × height preferably is at least 4.8 mm × 3.6 mm, in particular at least 5.7 mm × 4.2 mm, preferably at least 7.6 mm × 5.7 mm, preferably at least 8.8 mm × 6.6 mm, particularly at least 9.6 mm × 7.2 mm or at least 10.6 mm × 8.0 mm. Particularly, a size of the optical sensor of the camera in terms of width × height is from 4.8 mm × 3.6 mm to 13.2mm × 8.8 mm.

In general, the larger the sensor size, the more light can be captured by the sensor, which in turn improves the image quality. However, cameras with other sensor sizes might be suitable as well.

Additionally, supplementary lenses might be added to the camera, e.g. for extending or shortening the focal length of the camera.

Also, if available, additional internal wide angle and/or magnification lenses, both optical and/or digital, of the mobile computer device can be accesses on demand for taking the at least one digital image.

The predefined sample area can be a two-dimensional sample area or a three-dimensional sample area.

The two-dimensional sample area is a flat area, especially a flat rectangular area. The two-dimensional sample area is aligned horizontally and/or it is a horizontal sample area.

In the predefined sample area, especially the two-dimensional sample area, the solid samples preferably are arranged in a single layer and/or such that there is essentially no overlap of particles in the sample area.

However, according to another preferred implementation, the solid samples in the predefined sample area, especially in a three-dimensional sample area, are arranged in multiple layers and/or in a three-dimensional sample.

In this case, the method can for example be implemented such that only particles of the topmost surface layer and/or non-overlapping particles are considered in the at least one digital image whereas all other remaining particles are ignored for further processing. This can e.g. be achieved by appropriate image processing algorithms in step c).

Alternatively or in addition, image processing algorithms that are designed for identifying individual solid particles in particle agglomerates can be used in step c). These special measures will prevent that overlapping of solid particles is biasing the analysis.

Preferably, the predefined sample area, especially the two-dimensional sample area, comprises a reference scale and/or has a known size. A reference scale might for example be a character, a geometrical form, a ruler and/or a reference object of known size in the sample area. This allows for a precise determination of the size of the predefined sample area and a precise extraction of particle size parameters in step c).

Preferably, the mobile computer device is configured for automatically determining the size of the predefined sample area, especially the two-dimensional sample area, based on the reference scale.

In addition or alternatively, the predefined sample area, especially the two-dimensional sample area, has a predefined known size. In this case, preferably, the mobile computer device is configured for setting a predefined size, e.g. from a list of predefined sizes. In this case, no reference scale is required.

Especially, a thin sheet material, preferably of predefined size, is used as the predefined sample area, especially the two-dimensional sample area. For example a sheet of paper, e.g. a DIN A5, A4, or A3 paper, is used as the thin sheet material. Also, papers with other formats, such as e.g. tabloid, letter or statement format, can be used. Additionally, a reference scale can be present on the thin sheet material. Preferably, the mobile computer device is configured for automatically determining the size of the predefined sample area, especially the two-dimensional sample area.

Papers as predefined sample areas are readily available and rather cheap.

In general, the size of the predefined sample area, especially the two-dimensional sample area, affects the minimum identifiable particle size. The smaller the size of the sample area, the smaller solid particles can be identified in the predefined sample area, especially the two-dimensional sample area. Thus, for characterizing small particles, small predefined sample areas, especially small two-dimensional sample areas, are beneficial. Small particles in particular are particles with a particle size D90 not higher than 0.5 mm, preferably not higher than 0.1 mm, especially no higher than 0.063 mm.

Especially, the predefined sample area, in particular the thin sheet material, has a specific color different from the color of the solid particles. This helps to identify individual solid particles in step c) because the grain dimensions and the identification of particles close to the limit of the camera resolution can be identified better. Thus, preferably, the imaging particle analysis comprises a step of subtracting a specific background color.

Especially, the specific color of the predefined sample area, especially the two-dimensional sample area, is white. This results in a high contrast when characterizing solid particles typically used in curable compositions, such as e.g. sand particles. However, for other particles, different specific colors of the predefined sample area might be preferable.

Preferably, the predefined sample area, especially the two-dimensional sample area, in particular the thin sheet material, is placed on a surface, which in all directions of space is larger than the predefined sample area, especially the two-dimensional sample area, and that has a color that is different than the color of the predefined sample area. In particular, the color of the surface is chosen to show a high contrast with regard to the predefined sample area, especially the two-dimensional sample area. For example, if the color of the predefined sample area is white, the color of the surface is black.

In these cases, the predefined sample area, especially the two-dimensional sample area, is fully surrounded by a frame of a different color. This helps to identify the predefined sample area, especially the two-dimensional sample area, in the digital image.

According to a further preferred embodiment, a light emitting luminous surface is used as the predefined sample area, especially the two-dimensional sample area. Thereby, the luminous surface in particular is illuminated with a light source such that the luminous surface emits light with a homogeneous light distribution over the whole surface.

Especially, a light table pad, in particular a light pad, is used as the predefined sample area. This is an advantageous possibility to provide a light emitting luminous surface. A light table comprises a flat and luminous surface to be oriented horizontally that is illuminated with a light source from the backside. Especially, the luminous surface consists of a translucent layer that is illuminated from the backside with the light source.

A light pad in particular is a thin light table which a thickness of less than 20% or less than 10% of the width and less than 20% or less than 10% of the length of the luminous surface. Light tables and light pads are known, e.g. in the field of graphics, and commercially available.

When using a light emitting luminous surface, especially a light table, as the sample area, the sample of solid particles is arranged on top of the luminous surface, especially on the frontside of the luminous surface.

Compared to other predefined sample areas, such as e.g. papers, luminous surfaces, especially light tables or light pads, are in particular beneficial since shadows of the sample particles can be omitted, the contrast can be increased, less artefacts are produced, better particle recognition, especially of bright particles and/or small particles, is possible, and the fit of calculated outlines can be improved which in turn increases accuracy of particle shape parameter and particle size determination. Overall, the accuracy of the results can be improved.

Especially, the luminous surface is illuminated such that it emits a color different than the color of the solid particles, preferably the luminous surface is illuminated such that it emits white light.

In particular, the light source comprises a source of withe light. Optionally the light source additionally comprises a source of light of a color different from white. In particular, the color of the light source is switchable between the different colors. Colors different than white enhance detection of whitish, bright sands.

In particular, the light source is an LED light source. Compared to other light sources, this allows for minimizing heat evolution on the translucent surface or in the sample area, respectively. This reduces the risk of thermally induced changes of the sample.

LED may cause temporal light modulation disturbances. Temporal light modulation is a change in the luminous quantity or spectral distribution of light over time. Such modulations may result in undesirable visual perceptions such as flickering, stroboscopic effects and phantom array effects. Such effects are also known as temporal light artefacts. Such effects are described for example by J.A. Veitch et al in "On the state of knowledge concerning the effects of temporal light modulation" 20 Lighting Res. Technol. 2021, 53, 89-92. It is preferable within the present context, to avoid such temporal light modulation and effects resulting therefrom. Thus, according to some embodiments, the light source is configured to avoid effects resulting from temporal light modulation.

Preferably, the light emitting luminous surface, especially a light table or a light pad, is configured such that the light intensity of the luminous surface can be adjusted, especially continuously or in discrete steps. For example, the light emitting luminous surface, especially the light table or the light pad, is configured such that the light intensity can be switched between 2 - 5, especially 3 - 4, different light intensities.

In a further preferred embodiment, the light emitting luminous surface, especially the light table or the light pad, is configured such that it emits polarized light. This can e.g. be achieved by using a light source producing polarized light and/or a polarization filter arranged behind, on top of and/or within the luminous surface. A polarization filter can e.g. be selected from a foil. Polarized light can be used to further enhance the determination of the particle parameters and shapes. A foil can also be a colored foil.

In further preferred embodiments, the emitted light is such that it does not cause interferences.

Furthermore, the luminous surface, especially of a light table or a light pad can, be covered with a protective foil, e.g. for increasing scratch resistance, whereby preferably the protective foil is transparent with respect to the emitted light of the luminous surface. In particular, the protective foil is made of synthetic material. Especially, the protective foil is a replaceable foil.

Particularly, the light emitting luminous surface, especially the light table or the light pad, comprises a frame surrounding the light emitting luminous surface, whereby, the frame has a color different than the light emitting luminous surface, especially a darker color than the light emitting luminous surface, in particular a black color. This helps to identify the predefined sample area, especially the two-dimensional sample area, in the digital image.

For example, as size of the light emitting luminous surface, especially of the light table or the light pad, is equal to the size of a DIN A5, A4, or A3 paper or has the size of the tabloid, letter or statement format. Typically, light pads are slightly bigger in size than any of the afore mentioned formats to ensure that a paper of a given format would fit perfectly to a light pad of such format. Thus, the actual size of the light emitting luminous surface, especially of the light table or the light pad, may also be slightly bigger than any of the afore mentioned formats. Preferably, the mobile computer device is configured for manually and/or automatically determining the size of the light emitting luminous surface.

Taking the at least one digital image of the sample of solid particles with the camera preferably is performed under daylight conditions and/or with a light source, e.g. a flashlight, for illuminating the solid particles. Thereby, the light used for illuminating the solid particles preferably is well dispersed in order to avoid shadows and light inhomogeneities. The mobile computer device preferably is configured for automatically adjusting light conditions in order to obtain a balanced exposure.

In a further preferred embodiment, the camera is aligned plane-parallel, especially horizontally, to the predefined sample area, especially the two-dimensional sample area. Preferably, the mobile computer device is configured for automatically warning the user and/or for preventing taking the at least one image as long as there is a non-plane-parallel alignment. In this case, the mobile computer device preferably comprises at least one position sensor which can be assessed when performing the inventive method.

Especially, the camera is aligned horizontally, in particular horizontally and plane-parallel to the predefined sample area, when taking the at least one digital image of the sample of solid particles in step b). Thereby the predefined sample area is a horizontal area and/or it is aligned horizontally. Horizontal alignment can be conducted manually or by using a supporting function that are known to the skilled person.

Especially, the camera is aligned horizontally if its optical axis runs vertically. The optical axis is an imaginary line that defines the path along which light propagates through the camera system.

Taking the at least one digital image of the sample of solid particles in step b) with the camera in horizontal alignment, in particular horizontally and plane-parallel to the predefined sample area, greatly simplifies the image capturing process. Specifically, by adjusting the height of the camera over the predefined sample area, a share of the sample area in the image can be easily maximized with the horizontal alignment.

Also, a horizontal alignment of the predefined sample area or a horizontal sample area is beneficial since the solid particles will automatically remain stable and motionless in their position during the image capturing process. Thereby, the predefined sample area can for example be located on a table or any other essentially horizontal surface.

With the camera in vertical alignment and a non-horizontal alignment of the sample area, this is less convenient and requires special measures to keep the solid particles of the sample in place.

However, the method can be implemented as well with a non-plane-parallel alignment of the camera.

In particular, when taking the at least one digital image of the sample of solid particles with a camera of a mobile computer device or a camera connected to a mobile device in step b), the solid particles of the sample remain motionless. This significantly improves the image quality and the imaging particle analysis in step c).

In a further preferred embodiment, at least two consecutive digital images of the sample area are taken. In this case, preferably, step c) is performed with the at least two digital images. This can be helpful for increasing the number of statistical counts and for more precisely determining the at least one particle size parameter and at least one particle shape parameter. In particular, in step c), the at least two digital images are superimposed.

Especially, when taking the at least one image, the camera is aligned so that a share of the sample area in the image is maximized. This can be implemented for example by providing alignment instructions to the user and/or by automatically adjusting at least one setting of the camera, e.g. the focal length of the camera. Thus, preferably, the mobile computer device is configured accordingly.

Highly preferred, a minimum detectable particle size is calculated, preferably by taking into account the resolution of the camera, the area share of the sample area in the total area of the image, and the real size of the sample area. Preferably, the mobile computer device is configured accordingly.

Especially, if the minimum detectable particle size is below a predetermined threshold, a warning is provided to the user, alignment instructions are provided to the user and/or a setting of the camera, e.g. the focal distance, is adjusted. The predetermined threshold can e.g. be set manually. This helps to avoid taking images under unsuitable conditions.

The imaging particle analysis of the at least one digital image in step c) can be implemented with known and readily available image analysis algorithms, e.g. by using software packages and/or libraries provided in Matlab (by MathWorks^{®}), OpenCV (cf. https://opencv.org), ImageJ (by Wayne Rasband; cf. https://imagej.net) and/or artificial intelligence algorithms.

The at least one particle size parameter extracted preferably comprises at least one of the following parameters:
- the particle size distribution of the population of particles identified in the at least one digital image;
- at least one statistical parameter selected from the group of average particle size, mean diameter, and/or Dₓ-value with x = 1 - 100, especially D₁₀, D₅₀, D₈₅ and D₁₀₀ values; and/or
- a deviation from a predefined nominal value and/or nominal distribution, e.g. from fuller curves, standard sieving curves for specific concrete types; and/or
- the fineness modulus.

According to especially preferred embodiments, the at least one particle size parameter extracted comprises or is the particle size distribution of the population of particles identified in the at least one digital image.

These are highly relevant parameters when providing formulations of curable compositions with solid particles. However, other parameters might be provided as well.

Thereby, the extracted particle size distribution in particular is meant to correspond to the particle size distribution as defined in standard EN 933-1:2012.

Another possible way to determine particle size distribution is laser light diffraction as described in ISO 13320:2009. Thus, the extracted particle size distribution in especially is meant to correspond to the particle size distribution as defined in standard ISO 13320:2009.

A Dₓ value has the meaning that a proportion of the given assembly of particles of x% has a lower particle size than the given value. Thus, a D₉₀ value, for example, means that 90% of the assembly of particles has a particle size smaller than the D₉₀ value given. Accordingly, the average particle size corresponds in particular to the D₅₀ value (50% of the particles are smaller than the given value, 50% are correspondingly bigger).

In general, the at least one particle size parameter extracted preferably comprises at least the particle size distribution of the population of particles identified in the at least one digital image.

Especially, for particle sizes below the minimum detectable particle size, the particle size distribution may be extrapolated based on the extracted particle size distribution. For example, polynomial extrapolation is used, especially based on Lagrange interpolation or using Newton's method of finite differences to create a Newton series that fits the extracted particle size distribution.

Especially, the at least one particle shape parameter extracted comprises at least one of the following parameters:
- roundness,
- sphericity,
- aspect ratio,
- roughness,
- solidity,
- flakiness index,
- shape index,
- percentage of crushed and broken surfaces, and/or
- angularity.

These are parameters that so far are hardly analyzed in daily work. However, they have significant influence for optimizing the grading curve of a curable composition.

Especially, the particle shape parameters are meant to correspond to the shape parameters as defined in standards EN 933-1:2012 to -7:2012.

Especially, the particle shape parameters include the aspect ratio expressed as the maximum Feret diameter to the minimum Feret diameter.

Further shape parameters which can be extracted are given in Blott and Pye, Sedimentology (2008) 55, 31-63.

In particular, the at least one particle shape parameter is extracted with regard to particles of a predetermined size only, especially to particles larger than a given threshold size. For relatively small particles, the particle shape of solid particles affects the properties of curable compositions less. For example, the at least one particle shape parameter is extracted with regard to particles > 0.5 mm, in particular > 1 mm.

Nevertheless, it is possible to analyze the shape parameters of all solid particles if desired.

Furthermore, it is possible to extract the at least one particle shape parameter with regard to particles larger than a given lower threshold size and particles smaller than a given upper threshold size. Thereby, in particular, at least two, at least three or more different particle shape parameters can be extracted simultaneously within the range between the lower threshold and the upper threshold.

This allows for identifying shape parameters of particles with specific sizes, which are for example known to affect certain properties of interest of curable compositions, such as e.g. the rheology of a curable composition.

According to another preferred implementation, for each of at least two or more predefined size fractions, e.g. sieve size fractions, of the particles, at least one individual particle shape parameter is extracted. Thereby, especially, at least one individual mean value of the at least one particle shape parameter is extracted for each size fraction. This allows for providing detailed information about the size dependent distribution of the particle shape parameters.

For example, a mean value of the at least one particle shape parameter is provided for each of the least two or more predefined particle size fractions, e.g. sieve size fractions, separately. Optionally, the particle counts per size fraction can be normalized by the volume of the particles considered, e.g. in order to obtain data comparable to particle size distributions. In addition, in this case, at least two, at least three or more different particle shape parameters can be provided simultaneously.

Such data can e.g. be presented in a bar plot with the predefined particle size fractions as categories and the corresponding mean values of the at least one particle shape parameters in the form of bars with heights or lengths proportional to the values that they represent.

Furthermore, it is possible to provide the mean value of the at least one particle shape parameter with regard to particles in several selected size fractions, e.g. particles being present in size fractions above and/or below a given fraction threshold. Similar to the above, this allows for identifying shape parameters of particles in size fractions, which are for example known to affect certain properties of interest of curable compositions.

In a further preferred implementation, for each of the at least one digital image, an outline image is generated. An outline image is also called a contour image and comprises the outlines of all of the identified particles in the digital image. The outline image can be made available in step d) via a user interface, via a machine interface and/or on a data storage medium. For example, the outline image can be displayed within the application and/or stored on an external server. The outline image can serve as tool to evaluate the quality of the digital image and/or the analysis performed.

Especially, in step b), at least two, preferably at least three, in particular at least five or at least ten, digital images are taken and for each image a particle analysis is performed in step c), and by taking into account each of the at least one particle size parameter and/or the at least one particle shape parameter individually extracted from the at least two images, a deviation, especially the standard deviation, of the at least one particle size parameter and/or the at least one particle shape parameter is determined. The deviation can serve as tool to evaluate the quality of the digital images and/or the analysis performed.

Especially, if a deviation is above a predetermined threshold, a warning can be provided to the user and/or if a deviation is above a predetermined threshold, a digital image and/or an outline image giving rise to diverging parameters might be identified and/or indicated.

Preferably, the inventive method further comprises the step of assigning at least one attribute of the sample of solid particles. Especially, the attribute is selected from:
- a unique identifier of the sample of solid particles;
- a chemical and/or physical property of the sample of solid particles, especially
   ∘ the nature of the solid particles, e.g. sand, limestone and/or polymeric particles;
   ∘ the type of the solid particles, e.g. natural, crushed, manufactured, recycled and/or re-used solid particles;
   ∘ a maximum grain size of the solid particles;
   ∘ a minimum grain size of the solid particles;
   ∘ the state of the solid particles, e.g. wet or dry; and/or
   ∘ pretreatments received, e.g. untreated or washed.
- descriptive information of the sample of solid particles, especially
   ∘ the location of the source of the solid particles, e.g. manually or automatically by position sensors;
   ∘ the intended use, e.g. a project name and/or a customer name; and/or
   ∘ a general comment.

Especially, the "manufactured" particles are meant to be aggregates as defined e.g. in the Annual review 2018-2019 of the European Aggregates Association, on page 5.

Preferably, the mobile computer device is configured to query the at least one attribute of the sample of solid particles attributes, especially before, during and/or after steps a) to b).

Especially, a unique identifier of the sample of solid particles is generated automatically.

In particular, the method is at least partly, especially completely, performed on the mobile computer device.

In case the method is completely performed on the mobile computer device, the complete characterization can take place on the mobile computer device without requiring any communication network. Also, the at least one digital image,
preferably together with the at least one attribute can be stored on the mobile computer device, e.g. for sharing, recalling and/or further evaluation.

Preferably, the mobile computer device is configured such that the at least one digital image, preferably together with the at least one attribute can be transferred to an external device via communication means, e.g. a communication interface of the mobile device, for storing. Likewise, the mobile computer device preferably is configured for recalling the stored data from the external device. According to another preferred implementation, the image analysis in step c) and/or the making available in step d) is/are conducted on a separate computer device, e.g. on a server.

In this case, preferably, the at least one digital image, preferably together with the at least one attribute is transferred to the external device via communication means, e.g. a communication interface of the mobile device. Such a decentralized solution is beneficial since computationally intensive step c) can be performed on another device which in turn helps to increase runtime of the mobile computer device. Furthermore, steps c) and/or d) can be optimized by updating the software part on the side of the external device without the user having to update the software part on the mobile computer device.

Thereby, if there is no communication network available, the at least one digital image, preferably together with the at least one attribute, can be stored temporarily on the mobile computer device and transferred to the external device later on when the communication network is available.

In this case, the image, preferably together with the at least one attribute, is stored on an external computer device, e.g. a server, in particular for sharing, recalling and/or further evaluation.

In step d) the at least one particle size parameter and the at least one particle shape parameter are made available via a user interface, via a machine interface and/or on a data storage medium. If desired, the at least one digital image,
optionally together with the at least one attribute, can be made available in addition. However, this is not a requirement since for daily work the image as such is hardly important for a user.

If the data is made available via a user interface, this can be done directly on the mobile computer device and/or an external computer device. Thereby, for example the at least one particle size parameter and the at least one particle shape parameter are plotted in a graph, e.g. the particle size distribution, and/or
presented in an animated plot, e.g. in a roundness versus sphericity plot or the bar plot of mean particle size parameter versus sieve opening.

Making available the data via a machine interface allows for transferring the data to an external computer device, e.g. a server and/or a desktop computer.

Also it is possible to make available the data on a data storage medium, e.g. on an internal data storage medium of the mobile computer device, a storage device attached to the mobile computer device and/or on a storage device of an external computer.

Especially, the at least one particle size parameter and the at least one particle shape parameter, optionally together with the at least one attribute, and optionally with the at least one image, are written in a data file with predefined file format. E.g. the file format is chosen from json, csv, txt and/or pdf. However, other file formats can be used as well. Typically, the data file does not include the at least one image. This helps to reduce the file size.

In particular, the data file is transferred to another application, a further mobile computer device and/or an external computer device. Transfer can be effected by any kind of communication means, e.g. wireless communication and/or wired communication. This allows a user to share the characterization of the solid particles with other users, transfer it to another application for further evaluation and/or to a data storage server. Thus, preferably, the mobile computer device is configured for transferring a data file to another application, a further mobile computer device and/or an external computer device.

A size of the solid particles for example ranges from >0 to 125 mm, preferably from 10 µm to 32 mm, more preferably from 0.063 mm to 16 mm, especially from 0.1 mm to 2 mm. Such kind of particles are typically used in curable compositions. However, the method can be used for characterization of other particles as well.

In particular, the solid particles of the sample are selected from sand, aggregates, fibers and/or glass spheres. However, other solid particles can be used as well,
especially sand replacements, manufactured sands, crushed and/or recycled construction materials, bio aggregates, natural or synthetic fibers and/or polymeric particles.

A further aspect of the present invention is a method for providing a formulation of a curable composition comprising at least a binder and solid particles, whereby the method comprises the steps of (i) characterizing a sample of the solid particles with the method as described above and (ii) determining nature and/or proportion of at least one component of the formulation, especially of the solid particles and/or of an additive in the formulation, by taking into account the at least one particle size parameter and/or the at least one particle shape parameter. Thereby, the solid particles are different from the binder, especially the solid particles do not comprise or consist of any binder.

Especially, the formulation is made available via a user interface, via a machine interface and/or on a data storage medium.

This method for providing a formulation of a curable composition can for example be implemented in an independent software application or it may be included in the same software application which is configured for characterizing the sample of solid particles as described above.

Applications capable of calculating formulations of curable compositions are known to the skilled person. For example, an application called "Sika Mix Design App" suitable for calculation of concrete formulations is available from Sika Services AG, Switzerland. The application comprises a data base of known raw materials and the possibility to add new raw materials to the database, e.g. the solid particles as characterized with the present invention.

Also, the invention is concerned with a method for producing a curable composition comprising at least a binder and solid particles, whereby the method comprises the steps of characterizing a sample of solid particles with the method as described above and mixing the solid particles with the binder and any optional further component, whereby, preferably, the at least one particle size parameter and/or the at least one particle shape parameter are considered for determining nature and/or proportion of at least one of the components, especially of the solid particles and/or an additive, in the composition.

In these cases, the curable composition preferably is mineral binder composition, e.g. a mortar or a concrete composition.

The expression "mineral binder" means in particular a binder which in the presence of water reacts in a hydration reaction to give solid hydrates or hydrate phases. This can by way of example be a hydraulic binder (e.g. cement or hydraulic lime), a latently hydraulic binder (e.g. slag), a pozzolanic binder (e.g. fly ash), or a non-hydraulic binder (e.g. gypsum or white lime).

The entire mineral binder advantageously comprises a proportion of at least 5% by weight of the hydraulic binder, in particular at least 20% by weight, preferably at least 50% by weight, specifically at least 75% by weight. In another advantageous embodiment the mineral binder comprises at least 95% by weight of hydraulic binder, in particular cement.

The mineral binder in particular comprises a hydraulic binder, preferably cement. Particular preference is given to Portland cement, in particular of the type CEM I, II, III, or IV (in accordance with the standard EN 197-1). However, it can also be advantageous that the binder composition comprises, in addition or instead of a hydraulic binder, other binders. These are in particular latently hydraulic binders and/or pozzolanic binders. Examples of suitable latently hydraulic binders and/or pozzolanic binders are slag, fly ash, and/or silica dust. In one advantageous embodiment the mineral binder comprises from 5 to 95% by weight, in particular from 20 to 50% by weight, of latently hydraulic binders and/or pozzolanic binders.

In a special embodiment, the mineral binder comprises a mixture of calcined clay, limestone, and Portland cement.

The term "clay" refers to a solid material composed to at least 30 wt.-%, preferably to at least 35 wt.-%, especially to at least 75 wt.-%, each relative to its dry weight, of clay minerals. A calcined clay is a clay material that has been put to a heat treatment, preferably at a temperature between 500 - 900 °C, or in a flash calcination process at temperatures between 800 - 1100 °C. According to especially preferred embodiments of the present invention, the calcined clay is metakaolin.

In preferred embodiments of the present invention the chemical compositions of limestone and Portland cement are as defined in standard EN 197-1:2011. In the alternative, limestone may also stand for magnesium carbonate, dolomite, and or mixtures of magnesium carbonate, dolomite, and/or calcium carbonate. It is especially preferred that limestone within the present context is a naturally occurring limestone mainly consisting of calcium carbonate (typically calcite and/or aragonite) but typically also containing some magnesium carbonate and/or dolomite. Limestone may also be a naturally occurring marl.

According to preferred embodiments, Portland cement is of type CEM I. According to embodiments, the Portland clinker content in a Portland cement of the present invention is at least 35 w%, preferably at least 65 wt.-%, especially at least 95 wt.- %, each based on the total dry weight of the cement.

According to embodiments, the mineral binder comprises calcined clay, limestone, and Portland cement in the following weight ratios:
**P : CC** is from 33 : 1 to 1 : 1, preferably from 8 : 1 to 1 : 1,
**CC : L** is from 10 : 1 to 1 : 50 , preferably from 10 : 1 to 1 : 33, more preferably from 5 : 1 to 1 : 10, and
**P : L** is from 20 : 1 to 1 : 4, preferably from 5 : 1 to 1 : 1.

According to embodiments, the mineral binder consists to at least 65 wt.-%, preferably at least 80 wt.-%, more preferably at least 92 wt.-%, in each case relative to the total dry weight of the mineral binder, of calcined clay, limestone, and Portland cement.

According to embodiments of the present invention, a mineral binder comprises a mixture of
a) 25 - 100 mass parts of Portland cement (**P**),
b) 3 - 50 mass parts of calcined clay (**CC**), especially of metakaolin,
c) 5 - 100 mass parts of limestone (**L**).

The solid particles in these cases preferably are selected from aggregates such as sand, gravel, stone, powdered quartz, and/or limestone.

An additive can be selected from materials conventionally used, such as concrete plasticizers, for example lignosulfonates, sulfonated naphthalene-formaldehyde condensates, sulfonated melamine-formaldehyde condensates, or polycarboxylate ethers (PCE), accelerators; corrosion inhibitors; retarders; shrinkage reducers; antifoams, and/or foam-formers.

A still further aspect is directed to a system comprising a mobile computer device, and optionally an additional separate computer device, whereby the system comprises:
(i) means for carrying out steps a) to d), of the method as described above, and/or
(ii) means for carrying out at least the steps a) and b), especially steps a) and b) and d), of the method of claim 1, and means for transferring at least one digital image of a sample of solid particles, optionally together with at least one attribute, to the separate computer device.

Another aspect of the invention is related to a system comprising a computer device, whereby the system comprises means for receiving at least one digital image of a sample of solid particles and means for carrying out at least steps c) and/or d) of the method as described above.

Furthermore, the invention is concerned with a computer-readable medium comprising instructions which, when executed by a mobile computer device, cause the mobile computer device to carry out at least the steps a) and b), in particular a) and b) and d), especially steps a) to d), of the method as described above.

Also the invention is related to a computer-readable medium comprising instructions which, when executed by a computer device, cause the computer device to receive at least one digital image, optionally together with at least one attribute, and perform at least steps c) and/or d) of the method as described above.

Another solution of the present invention, which can be implemented independently of the above described first solution, is described in the following.

A first aspect of the second solution is directed to a method for providing a formulation of a curable composition comprising at least a binder and solid particles, whereby the method comprises the steps of (i) obtaining at least one particle size parameter and/or at least one particle shape parameter of the solid particles and (ii) determining nature and/or proportion of at least one component of the formulation, especially of the solid particles and/or of an additive in the formulation, by taking into account the at least one particle size parameter and the at least one particle shape parameter.

Likewise, a further aspect of the second solution is related to a method for producing a curable composition comprising at least a binder and solid particles, whereby the method comprises the steps of (i) obtaining at least one particle size parameter and/or at least one particle shape parameter of the solid particles and (ii) mixing the solid particles with the binder and any optional further component, whereby, preferably, the at least one particle size parameter and the at least one particle shape parameter are considered for determining nature and/or proportion of at least one of the components, especially of the solid particles and/or an additive, in the composition.

In these two aspects of the second solution, the at least one particle size parameter and the at least one particle shape parameter of the solid particles can be obtained by a method, which is different to the above described method or the method of claim 1, respectively. For example, these parameters can be obtained in a conventional manner, e.g. via sieving, laser diffraction and/or methods as described in standard EN 933-1:2012 to -7:2012, standard ISO 13320:2009 and/or Blott and Pye, Sedimentology (2008) 55, 31-63.

However, these parameters are used in combination for determining nature and/or proportion of at least one component of the composition, especially of the solid particles and/or of an additive in the composition. This helps to improve the properties of the curable composition, especially when using low-grade solid particles such as e.g. low-grade aggregates.

In particular, when determining nature and/or proportion of at least one component of the formulation or when mixing the solid particles with the binder and any **optional** further component in steps (ii) of the methods as described above, at least one particle size parameter is determined for each of two or more predefined particle size fractions, e.g. sieve size fractions.

Thereby, preferably, for each of the at least one particle size fraction, an individual weight parameter is assigned to the respective size fraction. The weight parameters can be used to consider a size dependence of the at least one particle shape parameter. For example, the shape of small aggregates usually is less relevant for achieving a desired property of a curable composition when compared with the shape of larger aggregates. Therefore, considering the size dependence of the at least one shape parameter allows for further optimizing the mix design of the curable composition with regard to the desired property.

For example, based on the size and shape of aggregates, e.g. sands, the nature and/or proportions of the individual components of a curable composition, e.g. a mortar or concrete composition, can be adjusted to achieve a desired property of the curable composition, such as e.g. a desired viscosity and/or flow property. Thereby, particle size distribution, sphericity, roundness and/ aspect ratio can be considered. In particular, the weight parameters for aggregates in a size fraction of < 0.5 mm is selected such that the shape parameter for this size fraction has less impact on the adjustment of nature and/or proportions of the individual components of the curable composition than the shape parameter of the aggregates with a size fraction ≥ 0.5 mm.

Binder, solid particles and additive preferably are selected as defined above in connection with the description of the mineral binder composition of the first solution. Also, the above described application called "Sika Mix Design App" can be used.

Especially, the formulation is made available with a computer device via a user interface, via a machine interface and/or on a data storage medium. Implementation of this feature can be effected as described above.

A further aspect of the second solution is a system comprising a computer device, especially a mobile computer device, whereby the system comprises means for carrying out step (ii) of the method as described above, especially steps (i) and (ii) of the method as described above.

Another aspect of the second solution is directed to a computer-readable medium comprising instructions which, when executed by a computer device, cause the computer device to perform step (ii) of the method as described above, especially steps (i) and (ii) of the method as described above.

Especially, any data processed and/or produced with the inventive methods is encrypted, especially such that only authorized users can access the original data. Likewise, any computer program and/or application implementing the inventive methods are encrypted, too. Methods for encryption are known to the skilled person.

Further advantageous configurations of the invention are evident from the exemplary embodiments.

### Brief description of the drawings

The drawings used to explain the embodiments show:
- Fig. 1: A flow chart of an inventive computer-implemented method;
- Fig. 2: A schematic overview of a system comprising means for carrying out the method of Fig. 1;
- Fig. 3: A schematic view of the second step of the method of Fig. 1 whereby a user (not shown) holding a smartphone is taking an image of a sample of solid particles of different sizes;
- Fig. 4: An example of the structure of a data file;
- Fig. 5: A flow chart of another computer-implemented method;
- Fig. 6: A bar plot of selected particle shape parameters (roundness, sphericity and aspect ratio) per particle sieve size fraction;
- Fig. 7: An outline image overlaid over a digital image;
- Fig. 8: A schematic view of a light pad;
- Fig. 9: A comparison between a sample of particles on the non-illuminated surface of the light pad of Fig. 8 (left side) and the same sample of particles on the illuminated surface of the light pad of Fig. 8 (right side);
- Fig. 10: Another bar plot of selected particle shape parameters (roundness/R, sphericity/SP and aspect ratio/AR) per particle sieve size fraction together with the corresponding particle count per particle sieve size fractions with sizes > 0.5 mm.

### Exemplary embodiments

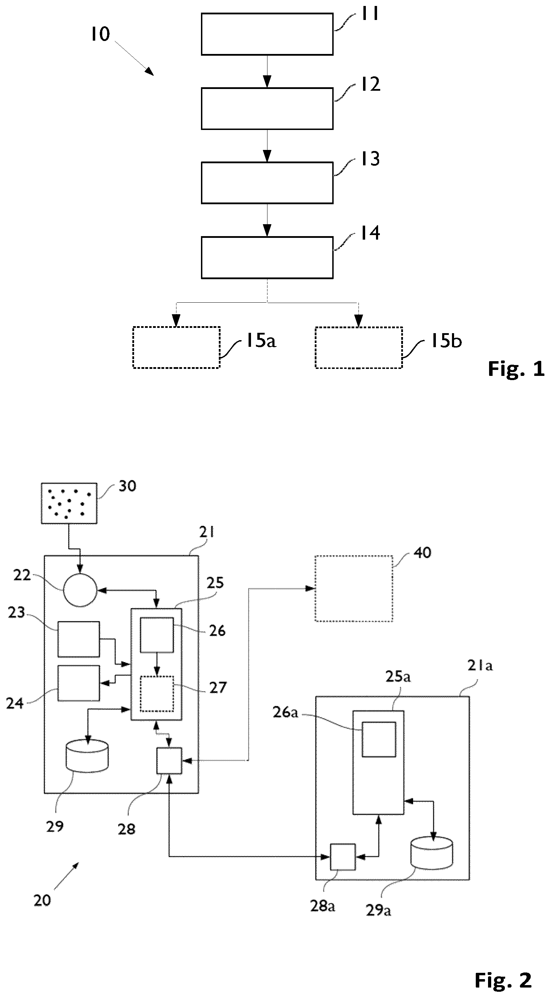

Fig. 1 shows a flow chart of an inventive computer-implemented method 10. In a first step 11, a sample of solid particles to be analyzed, e.g. a sand sample with particle sizes ranging from >0 to 2 mm, is provided on a two-dimensional sample area of known size. The sample area is for example formed by a sheet of paper of A4 size.

In a second step 12, a digital image of the sample of solid particles is taken with a camera of a mobile computer device, e.g. a smartphone. The camera for example has a 4K resolution.

Thereafter, in a third step 13, an imaging particle analysis of the digital image is performed for extracting at least one particle size parameter, e.g. the particle size distribution, and/or at least one particle shape parameter, e.g. roundness or sphericity, of the population of particles identified in the at least one digital image.

In a fourth step 14, the at least one particle size parameter and the at least one particle shape parameter are made available via a user interface, e.g. a display of the mobile computer device.

In an optional fifth step 15a, a formulation of a curable composition comprising at least a binder and solid particles is provided, whereby nature and/or proportion of at least one component of the formulation, especially an additive, e.g. a plasticizer, in the formulation, is determined by taking into account the at least one particle size parameter and the at least one particle shape parameter.

Alternatively or in addition, in an optional step 15b, a curable composition comprising at least a binder and solid particles is provided,
whereby step 15b comprises mixing the solid particles with the binder and any optional further component, whereby, the at least one particle size parameter and the at least one particle shape parameter are considered for determining nature and/or proportion of at least one of the components, especially of an additive, in the composition.

Fig. 2 shows a schematic overview of a system 20 comprising means for carrying out the method shown in Fig. 1.

Specifically, the system 20 comprises a smartphone 21 with a camera 22, a touch sensitive display comprising input device 23 and a display 24, a data processing unit 25 with a random access memory, a data storage device 29 and a wireless communication interface 28.

In operation, an application 26 is executed in the data processing unit 25, whereby the application is configured for performing steps 12 and 14 of the method described with Fig. 1.

Specifically, the application 26 assists a user in taking an image of solid particles in a two-dimensional sample area 30 with the built-in smartphone camera 22. Thereby, the application is for example configured for automatically warning the user and/or for preventing taking the image as long as there is a non-plane-parallel alignment. This can be achieved by assessing the position sensors of the smartphone (not shown). Also, the application is configured for automatically adjusting light conditions in order to obtain a balanced exposure. The digital image taken is stored in the random access memory and/or the data storage 29.

Additionally, the application 26 asks the user to enter one or more attributes of the sample via the input device and assigns a unique identifier to the sample. Attributes are e.g. the maximum grain size of the solid particles, the type of the solid particles (natural, crushed, manufactured, recycled; re-used solid particles), the state of the solid particles (wet or dry), the pretreatment (washed or untreated), the location of the source of the solid particles, the intended use (project name, customer name); and/or a general comment.

The query can e.g. be made by presenting to the user input fields, selection fields, maps and/or text input fields on the display 24 and storing the data provided by the user via the input device 23 together with the at least digital image in the random access memory and/or the data storage 29.

For example, the location of the source of particles can be provided manually by the user, e.g. by entering geo coordinates in input fields and/or by marking the position on a map shown on the display 24. It is however possible to automatically provide the location of the source of the solid particles e.g. by sensors of a global navigation satellite system, such as e.g. GPS, Galileo, Beidou and/or Glonass sensors. Thereby, the user might be requested to confirm the automatically determined position.

In the system 20 shown in Fig. 2, step 13 of the method shown in Fig. 1 is performed on an external server 21a. Specifically, the image taken with the smartphone camera 22, optionally together with the attributes, is transferred via the wireless communication interface 28 (or any other communication interface) and a network (e.g. the internet; not shown) to the server 21a. The server 21a receives the data via its communication interface 28a and forwards it to an imaging particle analysis application 26a running in a processing unit 25a.

The image, optionally together with the attributes can be stored on a data storage 29a of the server 21a for later sharing, recalling and/or further evaluation.

The application 26a performs an imaging particle analysis of the digital image whereby at least one particle size parameter, e.g. the particle size distribution, and at least one particle shape parameter, e.g. roundness or sphericity, of the population of particles identified in the at least one digital image is extracted. Thereby one or more attributes may be considered in the analysis as well.

The application 26a is implemented for example with image analysis algorithms such as e.g. software packages and/or libraries provided in Matlab, OpenCV and/or ImageJ, and/or with artificial intelligence software.

After the image analysis is finished, the at least one particle size parameter, e.g. the particle size distribution, and at least one particle shape parameter, e.g. roundness or sphericity, are sent back to the smartphone 21 or the application 25 being executed on it, respectively, via the communication interfaces 28a, 28.

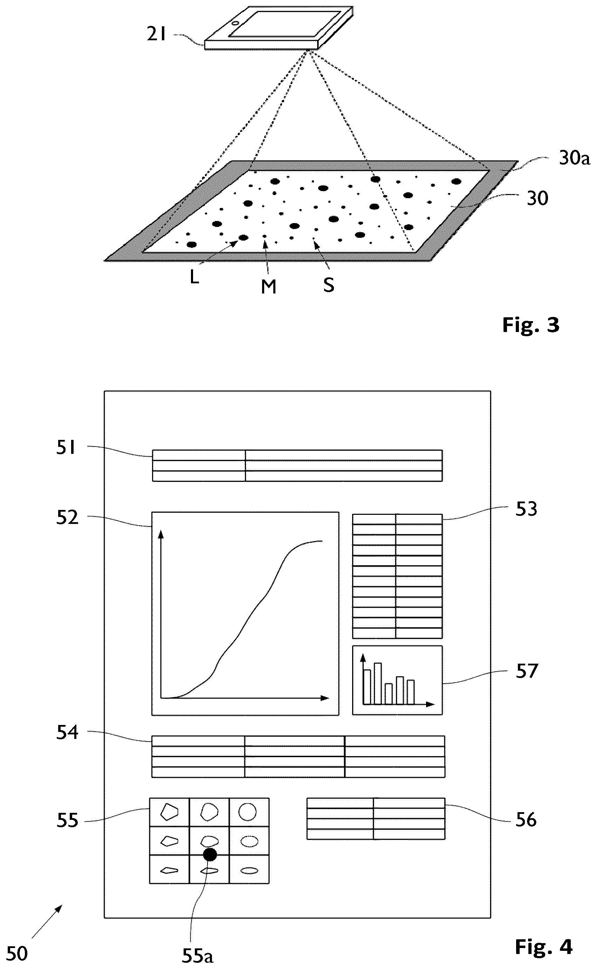

The application 25a then makes available the at least one particle size parameter and/or the at least one particle shape parameter via the display 24, or saves the parameters on the data storage 29, preferably together with the attributes, for later sharing, recalling and/or further evaluation. This data can be stored for example in the form of a data file having a file format chosen from json, csv, txt, pdf, and/or a proprietary file format. Especially, a file format capable of being read by the application called "Sika Mix Design App" and/or any other additional application is chosen. See Fig. 4 for an example.

Additionally, the application 25 is configured for sharing the at least one particle size parameter and the at least one particle shape parameter and the attributes with another user by sending them, in particular as a data file, via the communication interface 28 to a further computer device 40, e.g. a smartphone of another user. This can for example be initiated by the user via input device 23, e.g. by pressing a button shown on the display 24.

Furthermore, the system 20 may comprise an optional mix design application 27, which can be executed with the processing unit 25. The mix design application is configured for providing a formulation of a curable composition comprising at least a binder and solid particles is provided, whereby nature and/or proportion of at least one component of the formulation, especially an additive, e.g. a plasticizer, in the formulation, is determined by taking into account the at least one particle size parameter and the at least one particle shape parameter obtained with the first application 26.

Thus, for example, the first application can forward the at least one particle size parameter and the at least one particle shape parameter, and optionally the attributes, to the mix design application 27 via an appropriate application interface. Also the data to be sent to the mix design application 27 can be provided and transferred to the mix design application 27 in the form of a data file. In addition or alternatively, the mix design application can be configured for loading this data from the data storage 29 and/or in the form of a data file.

The mix design application 27 is configured for making available the formulation provided on the display 24, sending it via the communication interface 28 to another computer system, e.g. server 21a, or to a further smartphone 40, and/or save it on the data storage 29 for later sharing, recalling and/or further evaluation.

Fig. 3 shows a schematic view of step 12 of the method shown in Fig. 1. Thereby, a user (not shown), holding a smartphone 21 in his hands, takes an image of a sample of solid particles L (large), M (medium), S (small) of different sizes, e.g. sand particles with particle sizes ranging from >0 to 2 mm, being provided on a white sheet of paper of A4 size serving as a two-dimensional sample area 30. The sheet of paper is placed on a surface with high contrast, e.g. black surface, 30a that in all directions of space is larger than the two-dimensional sample area 30. Thus, the two-dimensional sample area 30 is fully surrounded by a frame of a different color.

Fig. 4 shows an example of the structure of a data file 50 in pdf file format. The data file 50 comprises a table 51 with attributes provided by the user, e.g. the type of the solid particles (natural, crushed, manufactured, recycled; re-used solid particles), the state of the solid particles (wet or dry), the pretreatment (washed or untreated), the location of the source of the solid particles, the intended use (project name, customer name); and/or a general comment.

Also, the file 50 comprises a graph 52 representing the particle size distribution, a table 53 comprising the calculated sieve size pass rate of the solid particles and/or the calculated proportion of retained solid particles, and a table 54 with statistical parameters, such as D₁₀, D₅₀, D₈₅, and D₁₀₀ values as well as the fineness modulus of the solid particles analyzed.

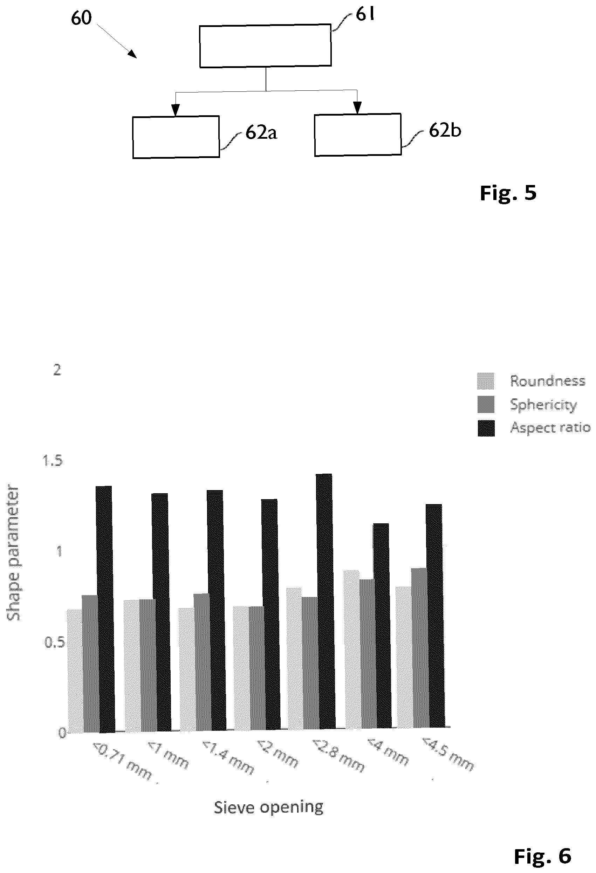

Furthermore, the file 50 comprises a two-dimensional plot 55 indicating the mean particle shape (for example roundness or sphericity) with a marker 55a. Additionally, the file 50 comprises a bar plot 57 displaying the mean value of selected particle shape parameters (for example roundness, sphericity and aspect ratio) per particle sieve size fraction. A more detailed view of the bar plot 57 is shown in Fig. 6.

Fig. 5 shows a flow chart of another computer-implemented method 60. Thereby, the particle size distribution and at least one particle shape parameter of a sample of solid particles are provided in a first step 61. These parameters can be obtained with the method shown in Fig. 1 or with any other method, e.g. manually.

Subsequently, in next step 62a, a formulation of a curable composition comprising at least a binder and solid particles is provided, whereby nature and/or proportion of at least one component of the formulation, especially an additive, e.g. a plasticizer, in the formulation, is determined by taking into account the particle size distribution and the at least one particle shape parameter.

This can be achieved with a mix design application as described above, i.e. mix design application 27. This application is configured for making available the formulation provided on the display 24, sending it via the communication interface 28 to another computer system, e.g. server 21a, or to a further smartphone 40, and/or save it on the data storage 29 for later sharing, recalling and/or further evaluation.

Alternatively or in addition, in an optional step 62b, a curable composition comprising at least a binder and solid particles is provided, whereby step 62b comprises mixing the solid particles with the binder and any optional further component, whereby, the at least one particle size parameter and/or the at least one particle shape parameter are considered for determining nature and/or proportion of at least one of the components, especially of an additive, in the composition.

As a representative example, a curable mortar composition comprising cement (CEM I), water, a plasticizer (Sika^{®} Viscocrete^{®} 111 P) and silica sand (D85-value = 2.36 mm) was produced as follows:
1. The particle size distribution as well as the sphericity, roundness and aspect ratio (= particle shape parameters) of the sand used were determined according to the method shown in Fig. 1.
2. The particle size distribution as well as the particle shape parameters were transferred to the "Sika Mix Design App" (available from Sika Services AG) for calculation of a mortar composition with a predefined slump flow of 370 mm (according to EN 12350-5:2019). Thereby, the proportions of cement, water, plasticizer and silica sand were adjusted to achieve the target slump flow.
3. Subsequently, the mortar composition as calculated by the "Sika Mix Design App" was produced by mixing the respective proportions of cement, water, plasticizer and silica sand. Then the slump flow of the so produced mortar composition was determined (according to EN 12350-5:2019) in order to compare the real slump flow of the mortar composition produced with the target slump flow of 370 mm.
4. For reasons of comparison, steps 2 and 3 were repeated without considering the particle shape parameters (sphericity, roundness and aspect ratio) in the "Sika Mix Design App" but otherwise identical conditions. Thus, in this case only the particle size distribution was considered in the "Sika Mix Design App" when calculating the mortar composition.

The results were as follows:

| | **Measured slump flow** | **Deviation from Target slump flow** |
|---|---|---|
| Target slump flow | 370 mm | - |
| Slump flow of composition calculated with particle size and particle shape parameters | 374 mm | 4 mm (+1.1%) |
| Slump flow of composition calculated without particle shape parameters | 346 mm | -24 mm (-6.5%) |

Thus, when considering the shape parameters, it is possible to predict a mortar composition that has a slump flow very close to the desired target value. Without the shape parameters, the deviation from the target slum flow is significantly higher.

Similar tests have been performed with other sands (not shown here). A statistical evaluation of the data has shown that considering the particle shape parameters for calculating a mortar composition having a desired target slump flow is highly relevant in general and results in significantly better predictions when compared with calculations that are not taking into account the shape parameters.

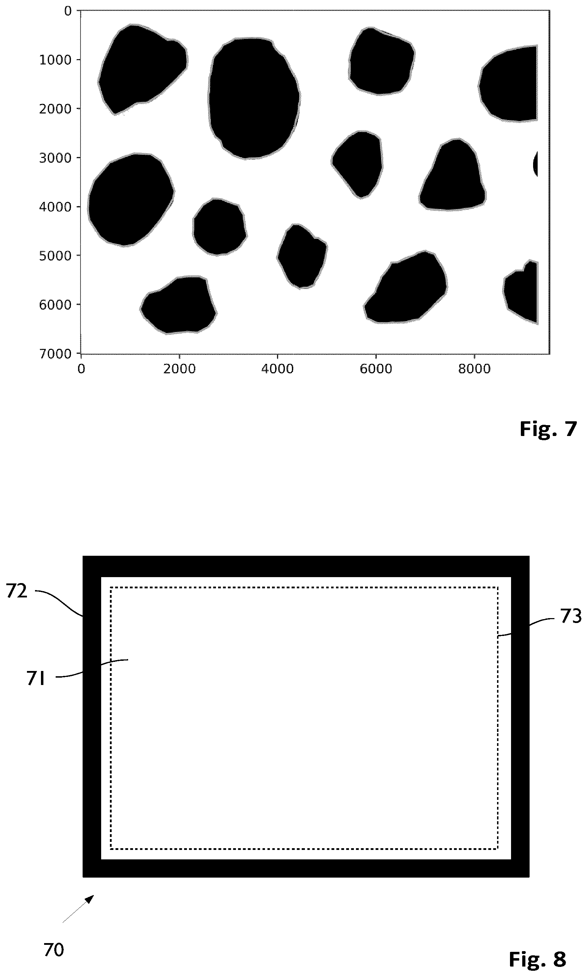

Fig. 7 shows an outline plot overlaid over the corresponding digital image. The outline plot can be used in addition to check the quality of the digital image and/or the analysis performed.

Fig. 8 shows a schematic view of a light pad 70 which can be used as the two dimensional sample area 30 instead of the sheet of paper used in the setup of Fig. 3. The light pad comprises of a light emitting luminous surface 71 made of a translucent layer that is illuminated with a white LED light source 73 (indicated by a dashed rectangle) from the backside. The light emitting luminous surface 71 has a size of a DIN A4 paper and is fully surrounded by a black frame 72.

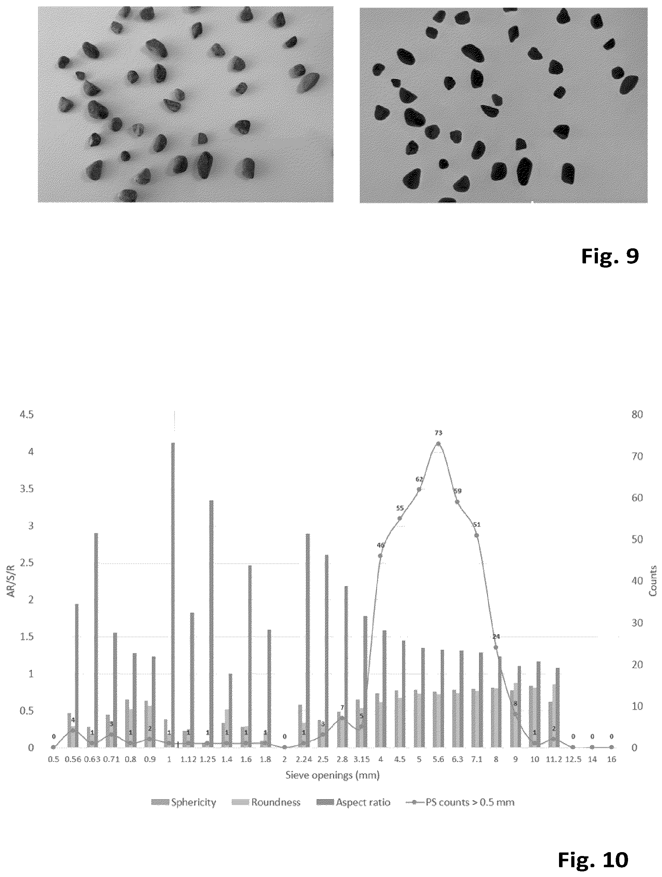

Fig. 9 shows a sample of particles on the non-illuminated surface of the light pad 70 of Fig. 8 (left side) and the same sample of particles on the illuminated surface of the light pad 70 of Fig. 8 (right side). As evident from the comparison, the contrast on the right side is clearly better and there are no shadows visible.

Fig. 10 shows an example of the particle counts of a sample of sand particles with a particle size > 0.5 mm per particle sieve size fractions (right axis), and additionally the corresponding mean values of three different particle shape parameters (roundness/R, sphericity/SP and aspect ratio/AR) per particle sieve size fraction (left axis).

The data shown in Fig. 10 was obtained with the method and the system described in Fig. 1 and 2. The application 26 used in this method may be further configured to calculate an overall mean value of the particle shape parameters for a selectable range of particle sieve size fractions, e.g. from a lower threshold of 4 mm to an upper threshold of 8 mm or alternatively from a lower threshold of 1 mm up the maximum sieve size fraction of 16 mm.

It will be appreciated by those skilled in the art that the present invention can be implemented in other specific forms without departing from the spirit or essential characteristics thereof. The presently disclosed implementations and embodiments are therefore considered in all respects to be illustrative and not restricted.

For example, instead of using server 21a, the application 26 can be configured as a standalone application capable of executing all of the steps 11, 12, 13, 14 and optionally 15a of the method of Fig. 1.

Likewise, it is possible to omit optional functions of the system 20, e.g. sharing of data with other computer devices, or adding additional functions, such as for example automatically retrieving of positional data via positioning sensors.

The method can as well be used for characterizing solid particles other than sands.

Also, it is possible to provide system 20 with mix design application 27 but without application 26. Such a system is for example suitable for performing the method as shown in Fig. 5. Thereby, the at least one particle size parameter and the at least one particle shape parameter can be inputted manually and/or being read from the data storage 29 and/or any other data storage, e.g. data storage 29a of server 21.

## Claims

1. Computer-implemented method for characterization of solid particles, especially sand particles, comprising the steps of:
a) Providing a sample of solid particles to be analyzed in a predefined sample area;
b) Taking at least one digital image of the sample of solid particles with a camera of a mobile computer device or a camera connected to a mobile device;
c) Performing an imaging particle analysis of the at least one digital image for extracting at least one particle size parameter and/or at least one particle shape parameter of the population of particles identified in the at least one digital image;
d) Making available the at least one particle size parameter and/or the at least one particle shape parameter via a user interface, via a machine interface and/or on a data storage medium,
**characterized in that** in step a) the sample area is a two-dimensional sample area which is aligned horizontally.

2. Method according to claim 1, whereby the mobile computer device comprises a human interface device, especially comprising an input device and a display, and, preferably a wireless communication interface.

3. Method according to any of preceding claims, whereby the mobile computer device is selected from a mobile phone, a mobile computer or a portable computer, and/or a head-mounted display with camera.

4. Method according to any of preceding claims, whereby the camera is a camera for taking images in the visible spectrum, especially color images.

5. Method according to any of preceding claims, whereby the camera has a resolution of at least 2 megapixels, especially at least 5 megapixels, preferably at least 8 megapixels, particularly at least 12 megapixels, highly preferred at least 20 megapixels, even more preferred at least 50 megapixels or at least more than 100 megapixels.

6. Method according to any of preceding claims, whereby the sample area comprises a reference scale and/or has a known size.

7. Method according to any of preceding claims, whereby the predefined sample area is a thin sheet material, especially of predefined size.

8. Method according to any of preceding claims, whereby the particles of the sample are selected from sand, aggregates, fibers and/or glass spheres.

9. Method according to any of preceding claims, whereby when taking the image, the camera is aligned so that a share of the sample area in the image is maximized, especially by providing alignment instructions to the user and/or by automatically adjusting at least one setting of the camera, e.g. the focal length of the camera.

10. Method according to any of preceding claims, whereby a minimum detectable particle size is calculated by taking into account the resolution of the camera, the length share of the sample area in the total area of the image, and the real length of the sample area.

11. Method according to any of preceding claims, whereby, if a minimum detectable particle size is below a predetermined threshold, a warning is provided to the user, alignment instructions are provided to the user and/or a setting of the camera, e.g. the focal distance, is adjusted automatically.

12. Method according to any of preceding claims, whereby the at least one particle size parameter extracted comprises the particle size distribution of the population of particles identified in the at least one digital image.

13. Method according to any of preceding claims, whereby for each of the at least one digital image, an outline image is generated, and, preferably, the outline image is made available in step d) via a user interface, via a machine interface and/or on a data storage medium.

14. Method according to any of preceding claims, whereby in step b), at least two, preferably at least three, in particular at least five or at least ten, digital images are taken and for each image an imaging particle analysis is performed in step c), and by taking into account each of the at least one particle size parameter and/or the at least one particle shape parameter individually extracted from the at least two images, a deviation, especially the standard deviation, of the at least one particle size parameter and/or the at least one particle shape parameter is determined.

15. Method according to claim 14, whereby, if the deviation is above a predetermined threshold, a warning is provided to the user and/or whereby a digital image and/or an outline image giving rise to diverging parameters is identified and/or indicated.

16. Method according to any of preceding claims, whereby the at least one particle size parameter comprises at least one statistical parameter selected from the group of average particle size, mean diameter, Dₓ-value with x = 1 - 100 and/or fineness modulus.

17. Method according to any of preceding claims, whereby the at least one particle size parameter extracted comprises a deviation from a predefined nominal value and/or nominal distribution.

18. Method according to any of preceding claims, whereby, for particle sizes below the minimum detectable particle size, the particle size distribution is extrapolated based on the extracted particle size distribution.

19. Method according to any of preceding claims, whereby the at least one particle shape parameter extracted comprises the roundness, sphericity, aspect ratio, roughness, solidity, flakiness index, shape index, percentage of crushed and broken surfaces, and/or angularity.

20. Method according to any of preceding claims, whereby the at least one particle shape parameter is extracted with regard to particles of a predetermined size only, especially to particles larger than a given threshold size, or whereby, for each of at least two or more predefined size fractions of the particles, at least one individual particle shape parameter is extracted, especially at least one individual mean value of the particle shape parameter is extracted.

21. Method according to any of preceding claims, comprising the step of assigning at least one attribute to the sample of solid particles.

22. Method according to any of preceding claims, whereby the method is at least partly, especially completely, performed on the mobile computer device.

23. Method according to any of preceding claims, whereby the image analysis in step c) and/or the making available in step d) is/are conducted on a separate computer device, e.g. on a server.

24. Method according to any of preceding claims, whereby the image, preferably together with the at least one attribute, and optionally the outline image, is stored on an external computer device, e.g. a server, in particular for sharing, recalling and/or further evaluation.

25. Method for providing a formulation of a curable composition comprising at least a binder and solid particles, whereby the solid particles are different from the binder, especially the solid particles do not comprise or consist of any binder, and whereby the method comprises the steps of (i) obtaining at least one particle size parameter and/or at least one particle shape parameter of the solid particles and (ii) determining nature and/or proportion of at least one component of the formulation, especially of the solid particles and/or of an additive in the formulation, by taking into account the at least one particle size parameter and the at least one particle shape parameter.

26. Method according to claim 29, whereby the formulation is made available with a computer device via a user interface, via a machine interface and/or on a data storage medium.

27. A system comprising a computer device, whereby the system comprises means for carrying out at least step (ii) of the method of claim 29, especially steps (i) and (ii) of the method of claim 29.

28. A computer-readable medium comprising instructions which, when executed by a computer device, cause the computer device to perform at least step (ii) of the method of claim 29, especially steps (i) and (ii) of the method of claim 29.

29. Method for producing a curable composition comprising at least a binder and solid particles, whereby the solid particles are different from the binder, especially the solid particles do not comprise or consist of any binder, and whereby the method comprises the steps of (i) obtaining at least one particle size parameter and/or at least one particle shape parameter of the solid particles and (ii) mixing the solid particles with the binder and any optional further component, whereby the at least one particle size parameter and the at least one particle shape parameter are considered for determining nature and/or proportion of at least one of the components, especially of the solid particles and/or an additive, in the composition.
